# EUROPEAN PATENT SPECIFICATION

(11) **EP 4 070 722 B1**
(45) Date of publication and mention of the grant of the patent: **25.09.2024**
(21) Application number: 20904868.5
(22) Date of filing: 30.10.2020
(51) Int. Cl.: A61B 5/0245

(54) **DIAGNOSTIC REPORT GENERATION COMPUTER PROGRAM AND APPARATUS**
COMPUTERPROGRAMM UND VORRICHTUNG ZUR ERZEUGUNG EINES DIAGNOSEBERICHTS
LOGICIEL ET APPAREIL DE GÉNÉRATION DE RAPPORT DE DIAGNOSTIC,

(30) Priority: 26.12.2019 CN 201911366780
(43) Date of publication of application: 12.10.2022
(73) Proprietor: Huawei Technologies Co., Ltd., Shenzhen, Guangdong 518129, (CN)
(72) Inventor: WANG, Shuo, Shenzhen, Guangdong 518129 (CN); YANG, Bin, Shenzhen, Guangdong 518129 (CN); CHEN, Yixin, Shenzhen, Guangdong 518129 (CN); REN, Huichao, Shenzhen, Guangdong 518129 (CN)
(74) Representative: Pfenning, Meinig & Partner mbB
(86) International application number: PCT/CN2020/125584
(87) International publication number: WO 2021/129152

(56) References cited:
- WO-A1-2019/210410
- CN-A- 103 815 893
- CN-A- 104 755 024
- CN-A- 106 456 032
- CN-A- 107 958 214
- CN-A- 108 652 635
- US-A- 5 284 152
- US-A1- 2009 171 227
- US-A1- 2010 145 205
- US-A1- 2014 142 448

## Description

### TECHNICAL FIELD

This application pertains to the field of physiological data processing technologies, and in particular, relates to a computer program, a diagnosis report generation apparatus, and a computer-readable storage medium.

### BACKGROUND

Document US 2014/142448 A1 discloses an apparatus and method for remotely managing a patient with a cardiac disease, including chronic cardiac diseases, and accurately managing arrhythmia of a patient.

With continuous development of science and technology, when a user wears a wearable device, heartbeat data may be monitored by using the wearable device. A terminal device may generate a diagnosis report based on collected heartbeat data, to notify the user whether an arrhythmia disease exists.

In a related technology, a wearable device may monitor a heartbeat of a user, and send detected electrocardiography (Electrocardiography, ECG) data to a terminal device connected to the wearable device. The terminal device may determine, based on the ECG data, whether the user has an arrhythmia disease to obtain a diagnosis result, and finally generate a diagnosis report based on a waveform (electrocardiogram) corresponding to the ECG data and the diagnosis result.

However, without professional knowledge, the user cannot determine a heartbeat waveform that indicates an arrhythmia disease in the electrocardiogram in the diagnosis report, resulting in a problem that readability of the diagnosis report is relatively low.

### SUMMARY

Embodiments of this application provide a computer program, a diagnosis report generation apparatus, and a computer-readable storage medium according to the independent claims, to resolve a problem of relatively low readability of a diagnosis report.

According to a first aspect, an embodiment of this application provides a computer program comprising instructions which, when the program is executed by a computer, cause the computer to carry out:
obtaining electrocardiography ECG data;
determining that the ECG data includes abnormal heartbeat data;
obtaining, based on the ECG data, an abnormal heartbeat waveform corresponding to the abnormal heartbeat data;
combining a normal heartbeat waveform and the abnormal heartbeat waveform to obtain an abnormality comparison image, where the abnormality comparison image displays a difference between the abnormal heartbeat waveform and the normal heartbeat waveform; and
generating a diagnosis report based on the abnormality comparison image.

Optionally, the obtaining, based on the ECG data, an abnormal heartbeat waveform corresponding to the abnormal heartbeat data includes:
determining whether heartbeat data in each heartbeat cycle in the ECG data is abnormal; and
for each heartbeat cycle, obtaining an abnormal heartbeat waveform in the heartbeat cycle if the heartbeat data in the heartbeat cycle is abnormal.

Optionally, the determining whether heartbeat data in each heartbeat cycle in the ECG data is abnormal includes:
obtaining a first prevalence probability of each of a plurality of arrhythmia diseases based on the ECG data;
obtaining a second prevalence probability of each of the plurality of arrhythmia diseases based on the heartbeat data in each heartbeat cycle;
performing calculation based on a plurality of first prevalence probabilities, a plurality of second prevalence probabilities, and preset probability weights, to obtain an overall prevalence probability of each arrhythmia disease; and
determining, based on a plurality of overall prevalence probabilities, whether the heartbeat data in each heartbeat cycle is abnormal.

Optionally, the obtaining a first prevalence probability of each of a plurality of arrhythmia diseases based on the ECG data includes:
extracting a feature between heartbeat data in all heartbeat cycles in the ECG data, to obtain first feature data; and
inputting the first feature data into a preset first classification model, to obtain the plurality of first prevalence probabilities; and
the obtaining a second prevalence probability of each of the plurality of arrhythmia diseases based on the heartbeat data in each heartbeat cycle includes:
   extracting a feature of the heartbeat data in each heartbeat cycle, to obtain second feature data of the heartbeat data in each heartbeat cycle; and
   inputting each piece of second feature data into a preset second classification model, to obtain the plurality of second prevalence probabilities in the heartbeat data in each heartbeat cycle.

Optionally, the computer program further causes the computer to carry out:
for each heartbeat cycle, adding a heartbeat waveform corresponding to the heartbeat cycle to a normal heartbeat waveform set if the heartbeat data in the heartbeat cycle is normal; and
generating the normal heartbeat waveform based on at least one heartbeat waveform in the normal heartbeat waveform set.

Optionally, the generating the normal heartbeat waveform based on at least one heartbeat waveform in the normal heartbeat waveform set includes:
obtaining feature data of each band of each heartbeat waveform in the normal heartbeat waveform set;
inputting the feature data of each band into a waveform correction model corresponding to each band, to obtain a standard waveform of each band; and
combining a plurality of standard waveforms, to obtain the normal heartbeat waveform.

Optionally, the computer program further causes the computer to carry out:
using a preset electrocardiogram waveform as the normal heartbeat waveform if the heartbeat data in each heartbeat cycle in the ECG data is abnormal.

Optionally, before the determining whether heartbeat data in each heartbeat cycle in the ECG data is abnormal, the computer program further causes the computer to carry out:
determining whether the ECG data is arrhythmia data; and
the determining whether heartbeat data in each heartbeat cycle in the ECG data is abnormal includes:
   if the ECG data is the arrhythmia data, determining whether the heartbeat data in each heartbeat cycle in the ECG data is abnormal.

Optionally, the combining a normal heartbeat waveform and the abnormal heartbeat waveform to obtain an abnormality comparison image includes:
aligning the abnormal heartbeat waveform with the normal heartbeat waveform based on a time axis; and
displaying the aligned abnormal heartbeat waveform and the aligned normal heartbeat waveform based on a same amplitude axis, to obtain the abnormality comparison image.

Optionally, after the generating a diagnosis report based on the abnormality comparison image, the computer program further causes the computer to carry out:
displaying the diagnosis report, where the diagnosis report includes a complete waveform diagram, the complete waveform diagram includes a heartbeat waveform in each heartbeat cycle in the ECG data, at least one abnormal heartbeat waveform is marked in the complete waveform diagram, and each abnormal heartbeat waveform corresponds to one abnormality comparison image; and
if an operation triggered for any abnormal heartbeat waveform in the complete waveform diagram is detected, displaying an abnormality comparison image corresponding to the abnormal heartbeat waveform, an arrhythmia disease corresponding to the abnormal heartbeat waveform, and an overall prevalence probability of the arrhythmia disease.

According to a second aspect, an embodiment of this application provides a diagnosis report generation apparatus, including:
a first obtaining module, configured to obtain electrocardiography ECG data, where the ECG data includes abnormal heartbeat data;
a second obtaining module, configured to obtain, based on the ECG data, an abnormal heartbeat waveform corresponding to the abnormal heartbeat data;
a combination module, configured to combine a normal heartbeat waveform and the abnormal heartbeat waveform to obtain an abnormality comparison image, where the abnormality comparison image displays a difference between the abnormal heartbeat waveform and the normal heartbeat waveform; and
a first generation module, configured to generate a diagnosis report based on the abnormality comparison image.

Optionally, the second obtaining module is further configured to: determine whether heartbeat data in each heartbeat cycle in the ECG data is abnormal; and for each heartbeat cycle, obtain an abnormal heartbeat waveform in the heartbeat cycle if the heartbeat data in the heartbeat cycle is abnormal.

Optionally, the second obtaining module is further configured to: obtain a first prevalence probability of each of a plurality of arrhythmia diseases based on the ECG data; obtain a second prevalence probability of each of the plurality of arrhythmia diseases based on the heartbeat data in each heartbeat cycle; perform calculation based on a plurality of first prevalence probabilities, a plurality of second prevalence probabilities, and preset probability weights, to obtain an overall prevalence probability of each arrhythmia disease; and determine, based on a plurality of overall prevalence probabilities, whether the heartbeat data in each heartbeat cycle is abnormal.

Optionally, the second obtaining module is further configured to: extract a feature between heartbeat data in all heartbeat cycles in the ECG data, to obtain first feature data; and input the first feature data into a preset first classification model, to obtain the plurality of first prevalence probabilities.

The second obtaining module is further configured to: extract a feature of the heartbeat data in each heartbeat cycle, to obtain second feature data of the heartbeat data in each heartbeat cycle; and input each piece of second feature data into a preset second classification model, to obtain the plurality of second prevalence probabilities in the heartbeat data in each heartbeat cycle.

Optionally, the apparatus further includes:
an addition module, configured to: for each heartbeat cycle, add a heartbeat waveform corresponding to the heartbeat cycle to a normal heartbeat waveform set if the heartbeat data in the heartbeat cycle is normal; and
a second generation module, configured to generate the normal heartbeat waveform based on at least one heartbeat waveform in the normal heartbeat waveform set.

Optionally, the second generation module is further configured to: obtain feature data of each band of each heartbeat waveform in the normal heartbeat waveform set; input the feature data of each band into a waveform correction model corresponding to each band, to obtain a standard waveform of each band; and combine a plurality of standard waveforms, to obtain the normal heartbeat waveform.

Optionally, the apparatus further includes:
a third generation module, configured to use a preset electrocardiogram waveform as the normal heartbeat waveform if the heartbeat data in each heartbeat cycle in the ECG data is abnormal.

Optionally, the apparatus further includes:
a determining module, configured to determine whether the ECG data is arrhythmia data; and
the second obtaining module is further configured to: if the ECG data is the arrhythmia data, determine whether the heartbeat data in each heartbeat cycle in the ECG data is abnormal.

Optionally, the combination module is configured to: align the abnormal heartbeat waveform with the normal heartbeat waveform based on a time axis; and display the aligned abnormal heartbeat waveform and the aligned normal heartbeat waveform based on a same amplitude axis, to obtain the abnormality comparison image.

Optionally, the apparatus further includes:
a first display module, configured to display the diagnosis report, where the diagnosis report includes a complete waveform diagram, the complete waveform diagram includes a heartbeat waveform in each heartbeat cycle in the ECG data, at least one abnormal heartbeat waveform is marked in the complete waveform diagram, and each abnormal heartbeat waveform corresponds to one abnormality comparison image; and
a second display module, configured to: if an operation triggered for any abnormal heartbeat waveform in the complete waveform diagram is detected, display an abnormality comparison image corresponding to the abnormal heartbeat waveform, an arrhythmia disease corresponding to the abnormal heartbeat waveform, and an overall prevalence probability of the arrhythmia disease.

According to a third aspect, an embodiment of this application provides a terminal device, including a memory, a processor, and the computer program according to any one of the implementations of the first aspect that is stored in the memory and that can be run on the processor. When executing the computer program, the processor performs the computer program according to any one of the implementations of the first aspect.

According to a fourth aspect, an embodiment of this application provides a computer-readable storage medium. The computer-readable storage medium stores the computer program according to any one of the implementations of the first aspect is implemented.

According to a fifth aspect, an embodiment of this application provides the computer program product according to the first aspect. When the computer program product runs on a terminal device, the terminal device is enabled to perform the computer program according to any one of the implementations of the first aspect.

Compared with the conventional technology, embodiments of this application have the following beneficial effects.

According to embodiments of this application, the ECG data including the abnormal heartbeat data is obtained. The abnormal heartbeat waveform corresponding to the abnormal heartbeat data is obtained based on the ECG data. The normal heartbeat waveform and the abnormal heartbeat waveform are combined to obtain the abnormality comparison image. Finally, the diagnosis report is generated based on the abnormality comparison image for displaying the difference between the abnormal heartbeat waveform and the normal heartbeat waveform. The abnormal heartbeat waveform is obtained by extracting and analyzing the ECG data, so that the abnormal heartbeat waveform can be combined and compared with the normal heartbeat waveform, to obtain the abnormality comparison image for generating the diagnosis report. When viewing the diagnosis report, a user can intuitively determine, based on the abnormality comparison image in the diagnosis report, the abnormal heartbeat waveform and the difference between the abnormal heartbeat waveform and the normal heartbeat waveform. This improves readability of the diagnosis report.

### BRIEF DESCRIPTION OF DRAWINGS

FIG. 1 is a schematic diagram of a system architecture of a diagnosis report generation system related to a diagnosis report generation method according to this application;
FIG. 2 is a block diagram of a partial structure of a mobile phone according to an embodiment of this application;
FIG. 3 is a schematic flowchart of a diagnosis report generation method according to this application;
FIG. 4 is a schematic flowchart of another diagnosis report generation method according to this application;
FIG. 5 is a schematic diagram of an abnormality comparison image according to this application;
FIG. 6 is a schematic diagram of an interface for displaying a diagnosis report according to this application;
FIG. 7 is a schematic diagram of an interface for displaying an abnormality comparison image according to this application;
FIG. 8 is a block diagram of a structure of a diagnosis report generation apparatus according to this application;
FIG. 9 is a block diagram of a structure of another diagnosis report generation apparatus according to this application;
FIG. 10 is a block diagram of a structure of still another diagnosis report generation apparatus according to this application;
FIG. 11 is a block diagram of a structure of still another diagnosis report generation apparatus according to this application; and
FIG. 12 is a block diagram of a structure of still another diagnosis report generation apparatus according to this application.

### DESCRIPTION OF EMBODIMENTS

In the following descriptions, to illustrate rather than limit, specific details such as a particular system structure and a technology are provided to make a thorough understanding of embodiments of this application. However, persons skilled in the art should know that this application may also be implemented in other embodiments without these specific details. In other cases, detailed descriptions of well-known systems, apparatuses, circuits, and methods are omitted, so that this application is described without being obscured by unnecessary details.

Terms used in the following embodiments are merely intended to describe specific embodiments, but are not intended to limit this application. Terms "one", "a", "the", "the foregoing", "this", and "the one" of singular forms used in this specification and the appended claims of this application are also intended to include forms like "one or more", unless otherwise specified in the context clearly. It should be further understood that, in embodiments of this application, "one or more" refers to one, two, or more, and the term "and/or" describes an association between associated objects, and indicates that three relationships may exist. For example, A and/or B may indicate the following cases: Only A exists, both A and B exist, and only B exists, where A and B may be singular or plural. The character "/" usually represents an "or" relationship between the associated objects.

A diagnosis report generation method provided in embodiments of this application may be applied to a terminal device such as a mobile phone, a tablet computer, a wearable device, a notebook computer, an ultra-mobile personal computer (ultra-mobile personal computer, UMPC), a netbook, or a personal digital assistant (personal digital assistant, PDA). A specific type of the terminal device is not limited in embodiments of this application.

For example, the terminal device may be a station (STAION, ST) in a WLAN, or may be a cellular phone, a cordless phone, a Session Initiation Protocol (Session Initiation Protocol, SIP) phone, a personal digital assistant (Personal Digital Assistant, PDA) device, a handheld device having a wireless communication function, a computing device, another processing device connected to a wireless modem, a computer, a laptop computer, a handheld communications device, a handheld computing device, or the like.

By way of example and not limitation, when the terminal device is a wearable device, the wearable device may alternatively be a generic term for wearable devices such as glasses, gloves, watches, clothes, and shoes that are developed based on intelligent design of daily wearing by using wearable technologies. The wearable device is a portable device that can be directly worn by a user or integrated into clothes or an accessory of the user. The wearable device is not only a hardware device, but also implements a powerful function through software support, data exchange, and cloud interaction. In a broad sense, the wearable intelligent devices include full-featured and large-sized devices that can implement complete or partial functions without depending on smartphones, such as smart watches or smart glasses, and devices that focus on only one type of application function and need to work with other devices such as smartphones, such as various smart bands or smart jewelry for monitoring physical signs.

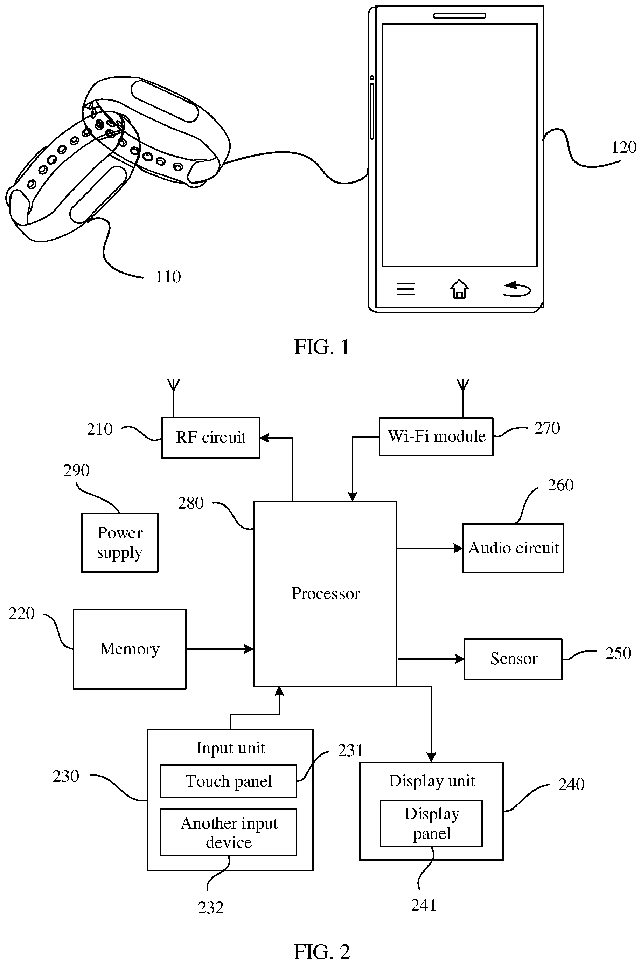

FIG. 1 is a schematic diagram of a system architecture of a diagnosis report generation system related to a diagnosis report generation method according to this application. As shown in FIG. 1, the diagnosis report generation system may include a wearable device 110 and a terminal device 120. The wearable device 110 is connected to the terminal device 120.

When a user wears the wearable device 110, the wearable device 110 monitors a heartbeat of the user, and records obtained ECG data. The terminal device 120 is configured to generate a diagnosis report of the user based on the ECG data monitored and sent by the wearable device 110. For example, the terminal device 120 may generate the diagnosis report of the user based on the ECG data in combination with a preset application.

In a possible implementation, when the user wears the wearable device 110, the wearable device 110 may collect the ECG data of the user, and send the collected ECG data to the terminal device 120.

Correspondingly, the terminal device 120 may receive the ECG data, analyze the ECG data to obtain an abnormal heartbeat waveform and a normal heartbeat waveform in the ECG data, then combine the abnormal heartbeat waveform and the normal heartbeat waveform, and display the abnormal heartbeat waveform and the normal heartbeat waveform in a same image, so as to generate an abnormality comparison image. Finally, a diagnosis report may be generated based on the abnormality comparison image.

It should be noted that the heartbeat waveforms indicated by the abnormal heartbeat waveform and the normal heartbeat waveform may be waveforms displayed in an electrocardiogram, or electrocardiogram waveforms. In embodiments of this application, information indicated by an electrocardiogram, an electrocardiogram waveform, and a heartbeat waveform is consistent.

For example, the terminal device is a mobile phone. FIG. 2 is a block diagram of a partial structure of a mobile phone according to an embodiment of this application. As shown in FIG. 2, the mobile phone includes components such as a radio frequency (Radio Frequency, RF) circuit 210, a memory 220, an input unit 230, a display unit 240, a sensor 250, an audio circuit 260, a wireless fidelity (wireless fidelity, Wi-Fi) module 270, a processor 280, and a power supply 290. Persons skilled in the art may understand that the structure of the mobile phone shown in FIG. 2 does not constitute a limitation on the mobile phone, and the mobile phone may include more or fewer components than those shown in the figure, or some components may be combined, or different component arrangements may be used.

The following specifically describes the components of the mobile phone with reference to FIG. 2.

The RF circuit 210 may be configured to: receive and send signals during information receiving and sending or during a call; particularly, after receiving downlink information of a base station, send the downlink information to the processor 280 for processing; and send uplink data to the base station. Usually, the RF circuit includes but is not limited to an antenna, at least one amplifier, a transceiver, a coupler, a low noise amplifier (Low Noise Amplifier, LNA), a duplexer, and the like. In addition, the RF circuit 210 may further communicate with a network and another device through wireless communication. The foregoing wireless communication may use any communications standard or protocol, which includes but is not limited to a Global System for Mobile Communications (Global System of Mobile communication, GSM), a general packet radio service (General Packet Radio Service, GPRS), Code Division Multiple Access (Code Division Multiple Access, CDMA), Wideband Code Division Multiple Access (Wideband Code Division Multiple Access, WCDMA), Long Term Evolution (Long Term Evolution, LTE), an email, a short message service (Short Messaging Service, SMS), and the like.

The memory 220 may be configured to store a software program and a module. The processor 280 performs various function applications of the mobile phone and data processing by running the software program and the module that are stored in the memory 220. The memory 220 may mainly include a program storage area and a data storage area. The program storage area may store an operating system, an application required by at least one function (such as a sound play function and an image play function), and the like. The data storage area may store data (such as audio data and a phone book) created based on use of the mobile phone, and the like. In addition, the memory 220 may include a high-speed random access memory, or may include a nonvolatile memory such as at least one magnetic disk storage device, a flash memory, or another volatile solid-state storage device.

The input unit 230 may be configured to: receive input digit or character information, and generate a key signal input related to a user setting and function control of the mobile phone. Specifically, the input unit 230 may include a touch panel 231 and another input device 232. The touch panel 231, also referred to as a touchscreen, may collect a touch operation of the user on or near the touch panel 231 (such as an operation of the user on or near the touch panel 231 by using any suitable object or accessory such as a finger or a stylus), and drive a corresponding connection apparatus based on a preset program. Optionally, the touch panel 231 may include two parts: a touch detection apparatus and a touch controller. The touch detection apparatus detects a touch location of the user, detects a signal brought by a touch operation, and delivers the signal to the touch controller. The touch controller receives touch information from the touch detection apparatus, converts the touch information into touch point coordinates, sends the touch point coordinates to the processor 280, and receives and executes a command sent by the processor 280. In addition, the touch panel 231 may be implemented in a plurality of types, such as a resistive type, a capacitive type, an infrared type, and a surface acoustic wave type. In addition to the touch panel 231, the input unit 230 may further include the another input device 232. Specifically, the another input device 232 may include but is not limited to one or more of a physical keyboard, a functional button (such as a sound volume control button or a power button), a trackball, a mouse, a joystick, and the like.

The display unit 240 may be configured to display information entered by the user or information provided for the user, and various menus of the mobile phone. The display unit 240 may include a display panel 241. Optionally, the display panel 241 may be configured in a form of a liquid crystal display (Liquid Crystal Display, LCD), an organic light-emitting diode (Organic Light-Emitting Diode, OLED), or the like. Further, the touch panel 231 may cover the display panel 241. After detecting a touch operation on or near the touch panel 231, the touch panel 231 transfers the touch operation to the processor 280, to determine a type of a touch event. Subsequently, the processor 280 provides a corresponding visual output on the display panel 241 based on the type of the touch event. Although in FIG. 2, the touch panel 231 and the display panel 241 serve as two independent components to implement input and input functions of the mobile phone. However, in some embodiments, the touch panel 231 and the display panel 241 may be integrated to implement the input and output functions of the mobile phone.

The processor 280 is a control center of the mobile phone and is connected to all parts of the entire mobile phone through various interfaces and lines, and performs various functions of the mobile phone and data processing by running or executing the software program and/or the module stored in the memory 220 and invoking data stored in the memory 220, to perform overall monitoring on the mobile phone. Optionally, the processor 280 may include one or more processing units. Preferably, an application processor and a modem processor may be integrated into the processor 280. The application processor mainly processes an operating system, a user interface, an application, and the like. The modem processor mainly processes wireless communication. It may be understood that the modem processor may not be integrated into the processor 280.

The mobile phone further includes the power supply 290 (such as a battery) that supplies power to all components. Preferably, the power supply may be logically connected to the processor 280 by using a power management system, to implement functions such as charging management, discharging management, and power consumption management by using the power management system.

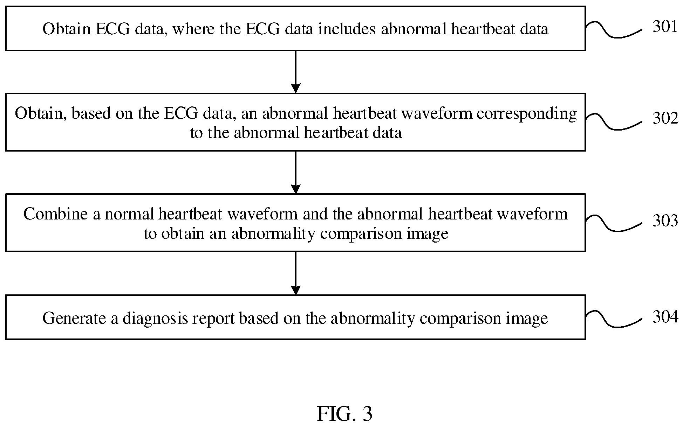

FIG. 3 is a schematic flowchart of a diagnosis report generation method according to this application. As an example instead of a limitation, the method may be applied to the foregoing terminal device. As shown in FIG. 3, the method includes the following steps.

S301: Obtain ECG data, where the ECG data includes abnormal heartbeat data.

The ECG data is data obtained by performing cardiac monitoring for a complete cycle. For example, the complete cycle of cardiac monitoring may be 30 seconds. In this case, the ECG data is ECG data that is of the complete cycle and that is obtained by performing cardiac monitoring for 30 seconds.

For user convenience of intuitively viewing a difference and a similarity between a heartbeat waveform corresponding to an arrhythmia disease and a normal heartbeat waveform, and determining an abnormal part of an electrocardiogram waveform, when a wearable device is worn, the wearable device may monitor a heartbeat of a user, and the terminal device may obtain the ECG data obtained through monitoring, so that in subsequent steps, a diagnosis report that is convenient for the user to view may be generated based on the ECG data.

It should be noted that, in actual application, the ECG data obtained through monitoring may include both normal heartbeat data and abnormal heartbeat data, or may all be normal heartbeat data, or may all be abnormal heartbeat data. In this embodiment of this application, that the ECG data includes at least one piece of abnormal heartbeat data is merely used as an example for description. The normal heartbeat data and the abnormal heartbeat data that are included in the ECG data are not limited in embodiments of this application.

S302: Obtain, based on the ECG data, an abnormal heartbeat waveform corresponding to the abnormal heartbeat data.

After obtaining the ECG data, the terminal device may first identify the ECG data of the complete cycle to determine the abnormal heartbeat data in the ECG data, and then obtain the corresponding abnormal heartbeat waveform based on the abnormal heartbeat data, so that in subsequent steps, a diagnosis report that is convenient to intuitively view may be generated based on the abnormal heartbeat waveform.

In a possible implementation, the terminal device may first simultaneously analyze heartbeat data in a plurality of heartbeat cycles in the ECG data of the complete cycle, and then separately analyze heartbeat data in each heartbeat cycle, so that the abnormal heartbeat waveform corresponding to the abnormal heartbeat data can be extracted by combining results of the two analyses.

It should be noted that not only the abnormal heartbeat waveform may be extracted based on the ECG data, but also the normal heartbeat waveform may be predicted based on the ECG data. If the normal heartbeat waveform cannot be obtained based on the ECG data, a preset electrocardiogram waveform may be used as the normal heartbeat waveform.

S303: Combine the normal heartbeat waveform and the abnormal heartbeat waveform to obtain an abnormality comparison image.

The abnormality comparison image displays a difference between the abnormal heartbeat waveform and the normal heartbeat waveform.

After at least one abnormal heartbeat waveform is extracted, each abnormal heartbeat waveform may be combined with the normal heartbeat waveform to obtain an abnormality comparison image corresponding to each abnormal heartbeat waveform, so that a difference between the abnormal heartbeat waveform and the normal heartbeat waveform is displayed through the abnormality comparison image. In this way, the user can intuitively view an abnormal band of the abnormal heartbeat waveform.

In a possible implementation, for each abnormal heartbeat waveform, the abnormal heartbeat waveform may be first aligned with the normal heartbeat waveform based on a time axis, so that the two waveforms can be compared based on a same moment. Then, the abnormal heartbeat waveform is projected onto the normal heartbeat waveform. In this way, the two waveforms are simultaneously displayed on a same time axis and a same amplitude axis, to obtain the abnormality comparison image corresponding to the abnormal heartbeat waveform.

S304: Generate a diagnosis report based on the abnormality comparison image.

After the abnormality comparison image corresponding to each abnormal heartbeat waveform is obtained, a diagnosis report may be generated based on the abnormality comparison image with reference to a complete waveform diagram corresponding to the ECG data of the complete cycle, so that when viewing the diagnosis report, the user can intuitively learn about the abnormal part of the electrocardiogram waveform based on the abnormality comparison image.

In a possible implementation, a heartbeat cycle in which each abnormal heartbeat waveform is located may be first determined in the complete waveform diagram based on the complete waveform diagram corresponding to the ECG data of the complete cycle, a correspondence between the complete waveform diagram and each abnormality comparison image is established based on the heartbeat cycle in which each abnormal heartbeat waveform is located, and then the diagnosis report is generated based on the correspondence with reference to an arrhythmia disease determined through analysis.

In conclusion, according to the diagnosis report generation method provided in this embodiment of this application, the ECG data including the abnormal heartbeat data is obtained. The abnormal heartbeat waveform corresponding to the abnormal heartbeat data is obtained based on the ECG data. The normal heartbeat waveform and the abnormal heartbeat waveform are combined to obtain the abnormality comparison image. Finally, the diagnosis report is generated based on the abnormality comparison image for displaying the difference between the abnormal heartbeat waveform and the normal heartbeat waveform. The abnormal heartbeat waveform is obtained by extracting and analyzing the ECG data, so that the abnormal heartbeat waveform can be combined and compared with the normal heartbeat waveform, to obtain the abnormality comparison image for generating the diagnosis report. When viewing the diagnosis report, the user can intuitively determine, based on the abnormality comparison image in the diagnosis report, the abnormal heartbeat waveform and the difference between the abnormal heartbeat waveform and the normal heartbeat waveform. This improves readability of the diagnosis report.

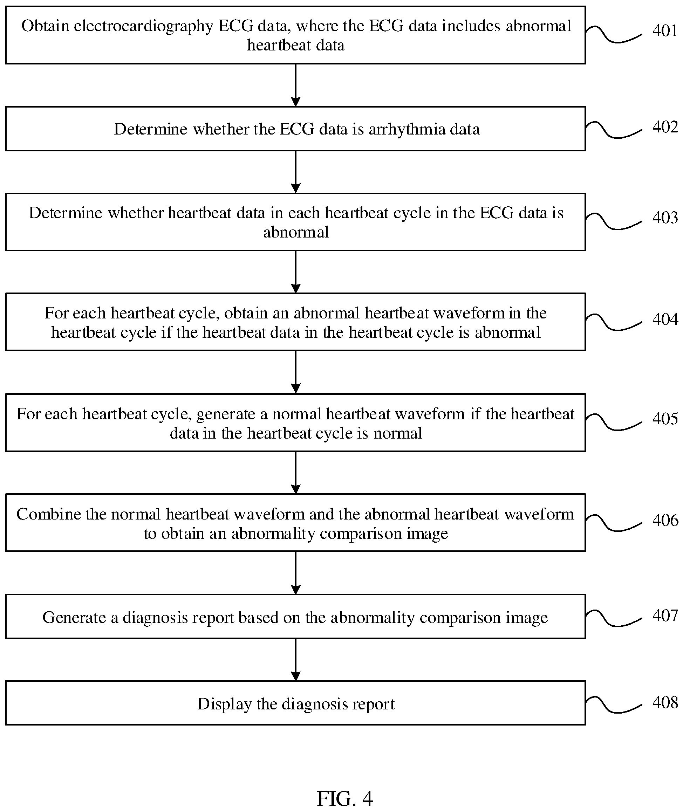

FIG. 4 is a schematic flowchart of another diagnosis report generation method according to this application. As an example instead of a limitation, the method may be applied to the foregoing terminal device. As shown in FIG. 4, the method includes the following steps.

S401: Obtain ECG data, where the ECG data includes abnormal heartbeat data.

S402: Determine whether the ECG data is arrhythmia data.

After obtaining the ECG data of a complete cycle, the terminal device may first perform preliminary determining based on the ECG data of the complete cycle, to determine whether an arrhythmia disease exists, so that in subsequent steps, different steps may be performed based on a determining result.

In a possible implementation, the terminal device may input the ECG data of the complete cycle into a pre-trained classification model, and analyze the ECG data of the complete cycle by using each neural network layer of the classification model, to determine whether the ECG data of the complete cycle is the arrhythmia data.

If the ECG data of the complete cycle is the arrhythmia data, S403 may be performed to determine, one heartbeat cycle by one heartbeat cycle, whether heartbeat data in each heartbeat cycle is abnormal. However, if the ECG data of the complete cycle is not the arrhythmia data, it indicates that neither the abnormal heartbeat data nor an abnormal heartbeat waveform exists in the ECG data of the complete cycle. In other words, no arrhythmia disease exists in the ECG data of the complete cycle.

The classification model may be a binary classification model, for identifying whether the abnormal heartbeat data exists in the ECG data of the complete cycle.

S403: Determine whether the heartbeat data in each heartbeat cycle in the ECG data is abnormal.

In all heartbeat cycles of the ECG data of the complete cycle, heartbeat data in any of a plurality of heartbeat cycles may be abnormal, or heartbeat data in each heartbeat cycle may be abnormal, or heartbeat data in each heartbeat cycle may be normal.

Therefore, the terminal device needs to determine whether the heartbeat data in each heartbeat cycle in the ECG data of the complete cycle is abnormal, so that in a subsequent step, an abnormal heartbeat waveform can be obtained based on the abnormal heartbeat data.

In a possible implementation, the terminal device may first simultaneously analyze and process heartbeat data in all the heartbeat cycles in the ECG data of the complete cycle, to determine a possible arrhythmia disease, and then perform one-by-one identification based on the heartbeat data in each heartbeat cycle, to determine whether the heartbeat data in each heartbeat cycle is abnormal, and whether an arrhythmia disease may exist, so as to comprehensively determine, based on two analysis results, whether the heartbeat data in each heartbeat cycle in the ECG data of the complete cycle is abnormal.

In addition, in a process of analyzing heartbeat data in the plurality of heartbeat cycles and a process of analyzing the heartbeat data in each heartbeat cycle one by one, different manners may be used for analysis.

Optionally, the terminal device may obtain a first prevalence probability of each of a plurality of arrhythmia diseases based on the ECG data; obtain a second prevalence probability of each of the plurality of arrhythmia diseases based on the heartbeat data in each heartbeat cycle; then perform calculation based on a plurality of first prevalence probabilities, a plurality of second prevalence probabilities, and preset probability weights, to obtain an overall prevalence probability of each arrhythmia disease; and finally determine, based on a plurality of overall prevalence probabilities, whether the heartbeat data in each heartbeat cycle is abnormal.

Further, in a process of obtaining the first prevalence probabilities, a feature between heartbeat data in all heartbeat cycles in the ECG data may be first extracted to obtain first feature data; and then the first feature data is input into a preset first classification model, to obtain the plurality of first prevalence probabilities.

Similarly, in a process of obtaining the second prevalence probabilities, a feature of the heartbeat data in each heartbeat cycle may be first extracted to obtain second feature data of the heartbeat data in each heartbeat cycle; and then each piece of second feature data is input into a preset second classification model, to obtain the plurality of second prevalence probabilities in the heartbeat data in each heartbeat cycle.

There may be a plurality of arrhythmia diseases. Therefore, in a process of determining a prevalence probability, a prevalence probability of each arrhythmia disease may be determined based on different diseases, to obtain the plurality of first prevalence probabilities and the plurality of second prevalence probabilities. For example, the arrhythmia diseases may include diseases that have an approximately normal heartbeat waveform in a single cycle, such as sinus arrhythmia and premature atrial contraction, and may also include diseases such as atrial fibrillation, atrioventricular block, and abnormal heart rhythm.

In a possible implementation, the terminal device may first extract a feature of the ECG data of the complete cycle to obtain the first feature data including data such as an RR interval average value, a variance, wavelet decomposition, and frequency band information; and input the extracted first feature data into the preset first classification model for analysis by using the first classification model; and finally obtain the first prevalence probability corresponding to each arrhythmia disease.

Then, the ECG data of the complete cycle may be segmented to obtain the heartbeat data in the plurality of heartbeat cycles, and then features of the heartbeat data in the plurality of heartbeat cycles may be extracted to obtain the second feature data including information such as an interval between a PR wave and an ST wave and a QST peak. Then, the second feature data is input into the second classification model, and a convolution analysis operation is performed by using the second classification model, to obtain the plurality of second prevalence probabilities corresponding to the heartbeat data in each heartbeat cycle.

Finally, calculation is performed based on the preset weights corresponding to the first prevalence probability and the second prevalence probability, the plurality of first prevalence probabilities, and the plurality of second prevalence probabilities, to obtain the overall prevalence probability of each arrhythmia disease corresponding to the heartbeat data in each heartbeat cycle. Then, each overall prevalence probability is compared with a probability threshold of the arrhythmia disease corresponding to the overall prevalence probability. If any overall prevalence probability is greater than the corresponding probability threshold, it may be determined that the heartbeat data in the heartbeat cycle corresponding to the overall prevalence probability is abnormal.

It should be noted that, in S403, whether the heartbeat data in each heartbeat cycle in the ECG data of the complete cycle is abnormal may be determined without performing S402, or S403 may be performed after S402 is performed and it is determined that the ECG data of the complete cycle is arrhythmia data. In other words, if the ECG data is the arrhythmia data, whether the heartbeat data in each heartbeat cycle in the ECG data is abnormal is determined.

S404: For each heartbeat cycle, obtain an abnormal heartbeat waveform in the heartbeat cycle if the heartbeat data in the heartbeat cycle is abnormal.

If it is determined in S403 that heartbeat data in a heartbeat cycle is abnormal, a heartbeat waveform corresponding to the heartbeat cycle may be obtained, and the heartbeat waveform corresponding to the heartbeat cycle is used as an abnormal heartbeat waveform. In addition, the abnormal heartbeat waveform corresponds to the heartbeat cycle, so that in subsequent steps, a diagnosis report may be generated based on an abnormal heartbeat waveform corresponding to each heartbeat cycle.

For example, a time period in which the heartbeat cycle is located may be first determined, the heartbeat waveform corresponding to the heartbeat cycle, that is, the abnormal heartbeat waveform, is obtained, and then a correspondence between the heartbeat cycle and the abnormal heartbeat waveform is established based on the time period.

S405: For each heartbeat cycle, generate a normal heartbeat waveform if the heartbeat data in the heartbeat cycle is normal.

Optionally, for each heartbeat cycle, if the heartbeat data in the heartbeat cycle is normal, a heartbeat waveform corresponding to the heartbeat cycle may be added to a normal heartbeat waveform set, and the normal heartbeat waveform is generated based on at least one heartbeat waveform in the normal heartbeat waveform set.

In a possible implementation, the terminal device may traverse the heartbeat data in each heartbeat cycle in the ECG data of the complete cycle. If heartbeat data in a heartbeat cycle is normal, a heartbeat waveform corresponding to the heartbeat cycle may be added to a preset normal heartbeat waveform set. After the traversing, the normal heartbeat waveform set that includes at least one heartbeat waveform may be obtained.

Then, the terminal device may extract a feature of each heartbeat waveform in the normal heartbeat waveform set, to obtain feature data of different bands, input the feature data of the different bands into waveform correction models corresponding to the bands, to obtain corrected waveforms of the bands, and finally perform stitching to obtain the normal heartbeat waveform.

Further, in a process of generating the normal heartbeat waveform, feature data of each band of each heartbeat waveform in the normal heartbeat waveform set may be obtained, the feature data of each band is input into a waveform correction model corresponding to each band to obtain a standard waveform, and then a plurality of standard waveforms are combined to obtain the normal heartbeat waveform.

In a possible implementation, the terminal device may extract an interval between a PR wave and an ST wave, a P wave, a QRS wave, and the PR wave of each heartbeat waveform in the normal heartbeat waveform set based on a preset waveform detection algorithm, then input each extracted band into a waveform correction model that matches the band, and perform regression prediction by using each waveform correction model and by using data such as a height and a width in the medical sense as penalty terms, so that a standard waveform of each band may be obtained. Then, standard waveforms of all bands may be combined, to obtain the normal heartbeat waveform.

For example, in a process of extracting a P wave, the terminal device may first detect a P wave of each heartbeat waveform in the normal heartbeat waveform set based on a P-wave waveform detection algorithm, to obtain a plurality of P waves through identification; input the P waves into a preset P-wave waveform correction model; perform regression prediction by using the P-wave waveform correction model and penalty terms such as a height and a width in the medical sense; and then correct a waveform obtained after the regression prediction based on feature information extracted from the plurality of P waves, to finally obtain standard waveforms of the P waves.

It should be noted that, if the heartbeat data in each heartbeat cycle in the ECG data is abnormal, it indicates that the normal heartbeat waveform cannot be obtained based on the ECG data of the complete cycle. In the case, a preset electrocardiogram waveform may be used as the normal heartbeat waveform.

S406: Combine the normal heartbeat waveform and the abnormal heartbeat waveform to obtain an abnormality comparison image.

The abnormality comparison image displays a difference between the abnormal heartbeat waveform and the normal heartbeat waveform.

After the normal heartbeat waveform and the at least one abnormal heartbeat waveform are obtained, the normal heartbeat waveform and the at least one abnormal heartbeat waveform may be compared, to generate an abnormality comparison image, so that in a subsequent step, a diagnosis report may be generated based on each abnormality comparison image.

Optionally, the abnormal heartbeat waveform may be aligned with the normal heartbeat waveform based on a time axis, and the aligned abnormal heartbeat waveform and the aligned normal heartbeat waveform may be displayed based on a same amplitude axis, to obtain the abnormality comparison image.

In a possible implementation, for each abnormal heartbeat waveform, each band in the abnormal heartbeat waveform may be obtained first. Then, each band in the normal heartbeat waveform is obtained. The first band in the abnormal heartbeat waveform is aligned with the first band in the normal heartbeat waveform, to obtain the aligned abnormal heartbeat waveform and the aligned normal heartbeat waveform.

Then, the aligned abnormal heartbeat waveform and the aligned normal heartbeat waveform may be simultaneously displayed on the same amplitude axis. In other words, both the aligned abnormal heartbeat waveform and the aligned normal heartbeat waveform are projected onto a same coordinate system. In this case, a projected image may be used as the abnormality comparison image.

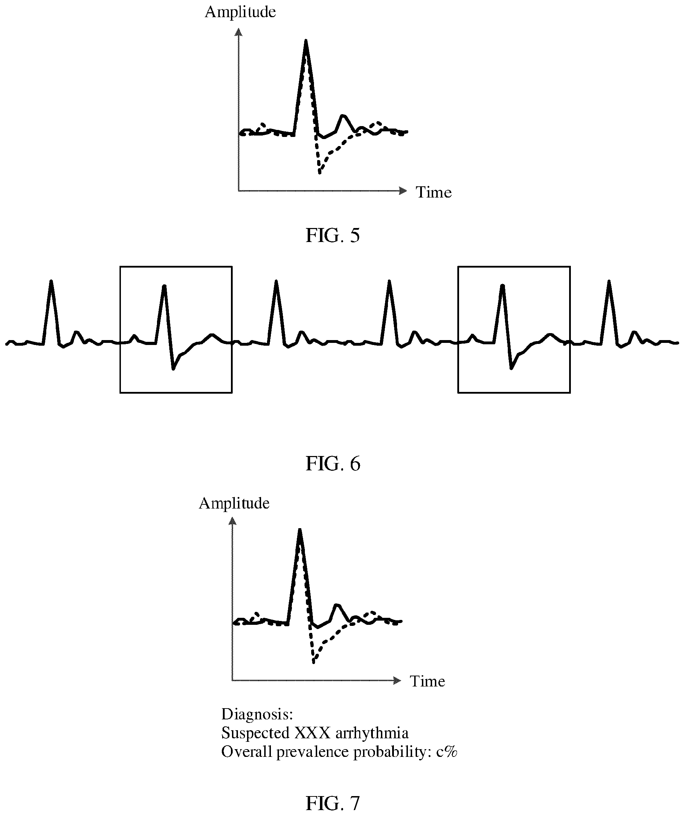

For example, FIG. 5 is a schematic diagram of an abnormality comparison image. In the figure, a solid line represents an aligned normal heartbeat waveform, and a dashed line represents an aligned abnormal heartbeat waveform. In this way, a difference between the normal heartbeat waveform and the abnormal heartbeat waveform can be intuitively viewed.

S407: Generate a diagnosis report based on the abnormality comparison image.

After the abnormality comparison image is obtained, the diagnosis report may be generated based on each abnormality comparison image, an arrhythmia disease corresponding to each abnormality comparison image, and a probability of each arrhythmia disease.

In a possible implementation, a complete waveform diagram may be obtained based on the ECG data of the complete cycle. In the complete waveform diagram, a heartbeat cycle in which each arrhythmia disease is located is selected, and a corresponding abnormal heartbeat waveform is determined based on the selected heartbeat cycle. Then, an abnormality comparison image generated by using the abnormal heartbeat waveform is determined, and a correspondence between the selected heartbeat cycle and the abnormality comparison image is established, to obtain the diagnosis report.

Further, in a process of establishing the correspondence between the selected heartbeat cycle and the abnormality comparison image, at least one arrhythmia disease and an overall prevalence probability corresponding to each arrhythmia disease that are determined in S403 may further be added to the abnormality comparison image.

For example, one or two arrhythmia diseases having the highest overall prevalence probability may be selected for addition.

S408: Display the diagnosis report.

After generating the diagnosis report, the terminal device may display the diagnosis report on a preset screen, and may detect an operation triggered by the user, and display, to the user, an abnormality comparison image corresponding to a heartbeat cycle in which each piece of abnormal heartbeat data is located.

Optionally, the terminal device may display the diagnosis report. If an operation triggered for any abnormal heartbeat waveform in the complete waveform diagram is detected, the terminal device displays an abnormality comparison image corresponding to the abnormal heartbeat waveform, an arrhythmia disease corresponding to the abnormal heartbeat waveform, and an overall prevalence probability of the arrhythmia disease.

The diagnosis report may include the complete waveform diagram. The complete waveform diagram may include a heartbeat waveform in each heartbeat cycle in the ECG data. At least one abnormal heartbeat waveform is marked in the complete waveform diagram. Each abnormal heartbeat waveform corresponds to one abnormality comparison image.

In a possible implementation, the terminal device may display a diagnosis report in which an abnormal heartbeat waveform is selected, and detect whether the user triggers a selection operation in an area in which each abnormal heartbeat waveform is located. If it is detected that the selection operation is triggered, a corresponding abnormal heartbeat waveform may be determined based on an area in which the selection operation is triggered, that is, a heartbeat cycle corresponding to the abnormal heartbeat waveform is determined, and then an abnormality comparison image corresponding to the heartbeat cycle is displayed based on a correspondence between the heartbeat cycle and the abnormality comparison image that is established when the diagnosis report is generated.

For example, as shown in FIG. 6 and FIG. 7, FIG. 6 shows an interface for displaying the diagnosis report by the terminal device. The interface includes a complete waveform diagram and an abnormal heartbeat waveform selected on the complete waveform diagram. FIG. 7 shows an interface for displaying the abnormality comparison image by the terminal device to the user. The interface includes a normal heartbeat waveform and an abnormal heartbeat waveform that are simultaneously displayed, a possible arrhythmia disease indicated by the abnormal heartbeat waveform, and an overall prevalence probability (c%, where c is a constant) corresponding to the arrhythmia disease.

In conclusion, according to the diagnosis report generation method provided in this embodiment of this application, the ECG data including the abnormal heartbeat data is obtained. The abnormal heartbeat waveform corresponding to the abnormal heartbeat data is obtained based on the ECG data. The normal heartbeat waveform and the abnormal heartbeat waveform are combined to obtain the abnormality comparison image. Finally, the diagnosis report is generated based on the abnormality comparison image for displaying the difference between the abnormal heartbeat waveform and the normal heartbeat waveform. The abnormal heartbeat waveform is obtained by extracting and analyzing the ECG data, so that the abnormal heartbeat waveform can be combined and compared with the normal heartbeat waveform, to obtain the abnormality comparison image for generating the diagnosis report. When viewing the diagnosis report, the user can intuitively determine, based on the abnormality comparison image in the diagnosis report, the abnormal heartbeat waveform and the difference between the abnormal heartbeat waveform and the normal heartbeat waveform. This improves readability of the diagnosis report.

It should be understood that sequence numbers of the steps in the foregoing embodiments do not mean execution sequences. The execution sequences of the processes should be determined based on functions and internal logic of the processes, and should not be constituted as any limitation on the implementation processes of embodiments of this application.

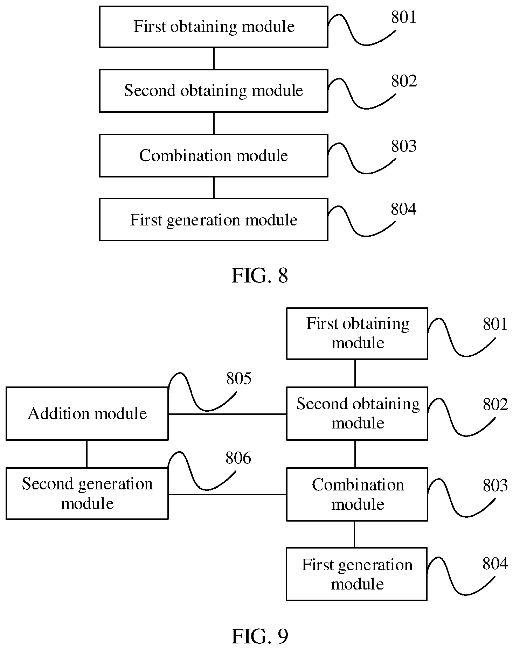

Corresponding to the diagnosis report generation method described in the foregoing embodiments, FIG. 8 is a block diagram of a structure of a diagnosis report generation apparatus according to this application. For ease of description, only a part related to this embodiment of this application is shown.

As shown in FIG. 8, the apparatus includes:
a first obtaining module 801, configured to obtain electrocardiography ECG data, where the ECG data includes abnormal heartbeat data;
a second obtaining module 802, configured to obtain, based on the ECG data, an abnormal heartbeat waveform corresponding to the abnormal heartbeat data;
a combination module 803, configured to combine a normal heartbeat waveform and the abnormal heartbeat waveform to obtain an abnormality comparison image, where the abnormality comparison image displays a difference between the abnormal heartbeat waveform and the normal heartbeat waveform; and
a first generation module 804, configured to generate a diagnosis report based on the abnormality comparison image.

Optionally, the second obtaining module 802 is further configured to: determine whether heartbeat data in each heartbeat cycle in the ECG data is abnormal; and for each heartbeat cycle, obtain an abnormal heartbeat waveform in the heartbeat cycle if the heartbeat data in the heartbeat cycle is abnormal.

Optionally, the second obtaining module 802 is further configured to: obtain a first prevalence probability of each of a plurality of arrhythmia diseases based on the ECG data; obtain a second prevalence probability of each of the plurality of arrhythmia diseases based on the heartbeat data in each heartbeat cycle; perform calculation based on a plurality of first prevalence probabilities, a plurality of second prevalence probabilities, and preset probability weights, to obtain an overall prevalence probability of each arrhythmia disease; and determine, based on a plurality of overall prevalence probabilities, whether the heartbeat data in each heartbeat cycle is abnormal.

Optionally, the second obtaining module 802 is further configured to: extract a feature between heartbeat data in all heartbeat cycles in the ECG data, to obtain first feature data; and input the first feature data into a preset first classification model, to obtain the plurality of first prevalence probabilities.

The second obtaining module 802 is further configured to: extract a feature of the heartbeat data in each heartbeat cycle, to obtain second feature data of the heartbeat data in each heartbeat cycle; and input each piece of second feature data into a preset second classification model, to obtain the plurality of second prevalence probabilities in the heartbeat data in each heartbeat cycle.

Optionally, as shown in FIG. 9, the apparatus further includes:
an addition module 805, configured to: for each heartbeat cycle, add a heartbeat waveform corresponding to the heartbeat cycle to a normal heartbeat waveform set if the heartbeat data in the heartbeat cycle is normal; and
a second generation module 806, configured to generate the normal heartbeat waveform based on at least one heartbeat waveform in the normal heartbeat waveform set.

Optionally, the second generation module 806 is further configured to: obtain feature data of each band of each heartbeat waveform in the normal heartbeat waveform set; input the feature data of each band into a waveform correction model corresponding to each band, to obtain a standard waveform of each band; and combine a plurality of standard waveforms, to obtain the normal heartbeat waveform.

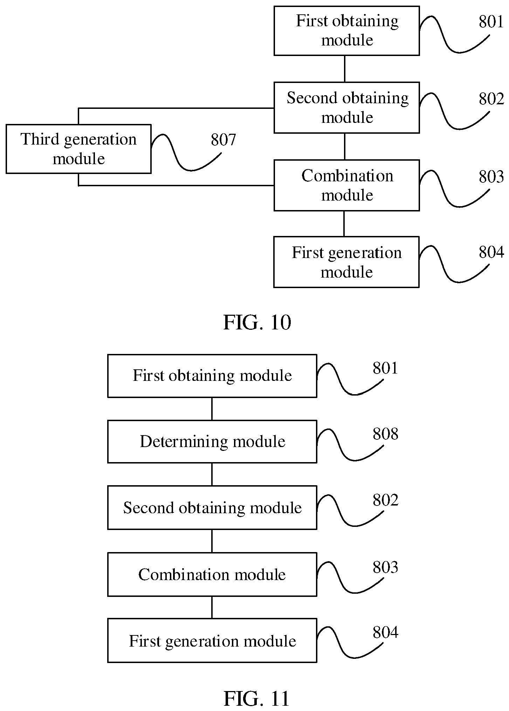

Optionally, as shown in FIG. 10, the apparatus further includes:
a third generation module 807, configured to use a preset electrocardiogram waveform as the normal heartbeat waveform if the heartbeat data in each heartbeat cycle in the ECG data is abnormal.

Optionally, as shown in FIG. 11, the apparatus further includes:
a determining module 808, configured to determine whether the ECG data is arrhythmia data; and
the second obtaining module 802 is further configured to: if the ECG data is the arrhythmia data, determine whether the heartbeat data in each heartbeat cycle in the ECG data is abnormal.

Optionally, the combination module 803 is configured to: align the abnormal heartbeat waveform with the normal heartbeat waveform based on a time axis; and display the aligned abnormal heartbeat waveform and the aligned normal heartbeat waveform based on a same amplitude axis, to obtain the abnormality comparison image.

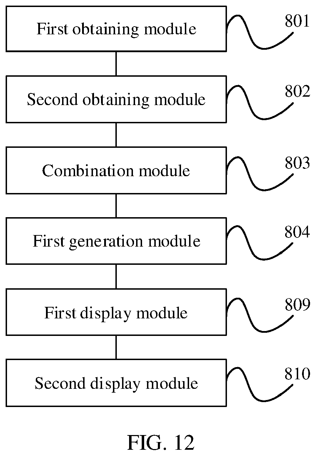

Optionally, as shown in FIG. 12, the apparatus further includes:
a first display module 809, configured to display the diagnosis report, where the diagnosis report includes a complete waveform diagram, the complete waveform diagram includes a heartbeat waveform in each heartbeat cycle in the ECG data, at least one abnormal heartbeat waveform is marked in the complete waveform diagram, and each abnormal heartbeat waveform corresponds to one abnormality comparison image; and
a second display module 810, configured to: if an operation triggered for any abnormal heartbeat waveform in the complete waveform diagram is detected, display an abnormality comparison image corresponding to the abnormal heartbeat waveform, an arrhythmia disease corresponding to the abnormal heartbeat waveform, and an overall prevalence probability of the arrhythmia disease.

In conclusion, according to the diagnosis report generation apparatus provided in embodiments of this application, the ECG data including the abnormal heartbeat data is obtained. The abnormal heartbeat waveform corresponding to the abnormal heartbeat data is obtained based on the ECG data. The normal heartbeat waveform and the abnormal heartbeat waveform are combined to obtain the abnormality comparison image. Finally, the diagnosis report is generated based on the abnormality comparison image for displaying the difference between the abnormal heartbeat waveform and the normal heartbeat waveform. The abnormal heartbeat waveform is obtained by extracting and analyzing the ECG data, so that the abnormal heartbeat waveform can be combined and compared with the normal heartbeat waveform, to obtain the abnormality comparison image for generating the diagnosis report. When viewing the diagnosis report, a user can intuitively determine, based on the abnormality comparison image in the diagnosis report, the abnormal heartbeat waveform and the difference between the abnormal heartbeat waveform and the normal heartbeat waveform. This improves readability of the diagnosis report.

Persons skilled in the art may clearly understand that, for the purpose of convenient and brief description, division into the foregoing function units or modules is merely used as an example for description. In an actual application, the foregoing functions may be allocated to different function units or modules for implementation according to a requirement. That is, an inner structure of the apparatus is divided into different function units or modules to implement all or some of the functions described above. Function units or modules in embodiments may be integrated into one processing unit, or each of the units may exist alone physically, or two or more units may be integrated into one unit. The integrated unit may be implemented in a form of hardware, or may be implemented in a form of a software function unit. In addition, specific names of the function units or modules are merely provided for distinguishing between the units or modules, but are not intended to limit the protection scope of this application. For a specific working process of the units or modules in the foregoing system, refer to a corresponding process in the foregoing method embodiments. Details are not described herein again.

In the foregoing embodiments, the description of each embodiment has respective focuses. For a part that is not described in detail in an embodiment, refer to related descriptions in other embodiments.

Persons of ordinary skill in the art may be aware that units and algorithm steps in the examples described with reference to embodiments disclosed in this specification may be implemented by electronic hardware or a combination of computer software and electronic hardware. Whether the functions are performed by hardware or software depends on particular applications and design constraint conditions of the technical solutions. Persons skilled in the art may use different methods to implement the described functions for each particular application, but it should not be considered that the implementation goes beyond the scope of this application.

In embodiments provided in this application, it should be understood that the disclosed apparatuses and methods may be implemented in other manners. For example, the described system embodiment is merely an example. For example, division into the modules or units is merely logical function division and may be other division in an actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, and may be located in one position, or may be distributed on a plurality of network units. Some or all of the units may be selected according to actual requirements to achieve the objectives of the solutions of embodiments.

In addition, function units in embodiments of this application may be integrated into one processing unit, or each of the units may exist alone physically, or two or more units may be integrated into one unit. The integrated unit may be implemented in a form of hardware, or may be implemented in a form of a software function unit.

When the integrated unit is implemented in the form of the software function unit and sold or used as an independent product, the integrated unit may be stored in a computer-readable storage medium. Based on such an understanding, in this application, all or some of the procedures of the methods in the foregoing embodiments may be implemented by a computer program instructing related hardware. The computer program may be stored in a computer-readable storage medium. When the computer program is executed by a processor, steps in the foregoing method embodiments can be implemented. The computer program includes computer program code, and the computer program code may be in a source code form, an object code form, an executable file form, some intermediate forms, or the like. The computer-readable medium may include at least the following: any entity or an apparatus capable of carrying computer program code to a terminal device, a recording medium, a computer memory, a read-only memory (ROM, Read-Only Memory), a random access memory (RAM, Random Access Memory), an electrical carrier signal, a telecommunications signal, and a software distribution medium, for example, a USB flash drive, a removable hard disk, a magnetic disk, or an optical disc. In some jurisdictions, according to legislation and patent practice, a computer-readable medium cannot be an electrical carrier signal or a telecommunications signal.

An embodiment of this application provides a terminal device, including a memory, a processor, and a computer program that is stored in the memory and that can be run on the processor. When executing the computer program, the processor implements the method shown in FIG. 3 or FIG. 4.

An embodiment of this application provides a computer-readable storage medium. The computer-readable storage medium stores a computer program, and when the computer program is executed by a processor, the methods shown in FIG. 3 and FIG. 4 are implemented.

## Claims

1. A computer program comprising instructions which, when the program is executed by a computer, cause the computer to carry out:
obtaining (301, 401) electrocardiography ECG data;
determining (402) that the ECG data comprises abnormal heartbeat data; and
obtaining (302), based on the ECG data, an abnormal heartbeat waveform corresponding to the abnormal heartbeat data;
**characterized in that** the computer program further causes the computer to carry out:
combining (303, 406) a normal heartbeat waveform and the abnormal heartbeat waveform to obtain an abnormality comparison image, wherein the abnormality comparison image displays a difference between the abnormal heartbeat waveform and the normal heartbeat waveform; and
generating (304, 407) a diagnosis report based on the abnormality comparison image.

2. The computer program according to claim 1, wherein the obtaining (302), based on the ECG data, an abnormal heartbeat waveform corresponding to the abnormal heartbeat data comprises:
determining (403) whether heartbeat data in each heartbeat cycle in the ECG data is abnormal; and
for each heartbeat cycle, obtaining (404) an abnormal heartbeat waveform in the heartbeat cycle if the heartbeat data in the heartbeat cycle is abnormal.

3. The computer program according to claim 2, wherein the determining (403) whether heartbeat data in each heartbeat cycle in the ECG data is abnormal comprises:
obtaining a first prevalence probability of each of a plurality of arrhythmia diseases based on the ECG data;
obtaining a second prevalence probability of each of the plurality of arrhythmia diseases based on the heartbeat data in each heartbeat cycle;
performing calculation based on a plurality of first prevalence probabilities, a plurality of second prevalence probabilities, and preset probability weights, to obtain an overall prevalence probability of each arrhythmia disease; and
determining, based on a plurality of overall prevalence probabilities, whether the heartbeat data in each heartbeat cycle is abnormal.

4. The computer program according to claim 3, wherein the obtaining a first prevalence probability of each of a plurality of arrhythmia diseases based on the ECG data comprises:
extracting a feature between heartbeat data in all heartbeat cycles in the ECG data, to obtain first feature data; and
inputting the first feature data into a preset first classification model, to obtain the plurality of first prevalence probabilities; and
the obtaining a second prevalence probability of each of the plurality of arrhythmia diseases based on the heartbeat data in each heartbeat cycle comprises:
extracting a feature of the heartbeat data in each heartbeat cycle, to obtain second feature data of the heartbeat data in each heartbeat cycle; and
inputting each piece of second feature data into a preset second classification model, to obtain the plurality of second prevalence probabilities in the heartbeat data in each heartbeat cycle.

5. The computer program according to claim 2, wherein the computer program further causes the computer to carry out:
for each heartbeat cycle, adding (405) a heartbeat waveform corresponding to the heartbeat cycle to a normal heartbeat waveform set if the heartbeat data in the heartbeat cycle is normal; and
generating (405) the normal heartbeat waveform based on at least one heartbeat waveform in the normal heartbeat waveform set.

6. The computer program according to claim 5, wherein the generating (405) the normal heartbeat waveform based on at least one heartbeat waveform in the normal heartbeat waveform set comprises:
obtaining feature data of each band of each heartbeat waveform in the normal heartbeat waveform set;
inputting the feature data of each band into a waveform correction model corresponding to each band, to obtain a standard waveform of each band; and
combining a plurality of standard waveforms, to obtain the normal heartbeat waveform.

7. The computer program according to claim 2, wherein the computer program further causes the computer to carry out:
using a preset electrocardiogram waveform as the normal heartbeat waveform if the heartbeat data in each heartbeat cycle in the ECG data is abnormal.

8. The computer program according to claim 2, wherein before the determining (403) whether heartbeat data in each heartbeat cycle in the ECG data is abnormal, the computer program further causes the computer to carry out:
determining whether the ECG data is arrhythmia data; and
the determining whether heartbeat data in each heartbeat cycle in the ECG data is abnormal comprises:
if the ECG data is the arrhythmia data, determining whether the heartbeat data in each heartbeat cycle in the ECG data is abnormal.

9. The computer program according to any one of claims 1 to 8, wherein the combining (303, 406) a normal heartbeat waveform and the abnormal heartbeat waveform to obtain an abnormality comparison image comprises:
aligning the abnormal heartbeat waveform with the normal heartbeat waveform based on a time axis; and
displaying the aligned abnormal heartbeat waveform and the aligned normal heartbeat waveform based on a same amplitude axis, to obtain the abnormality comparison image.

10. A diagnosis report generation apparatus, comprising:
a first obtaining module (801), configured to obtain electrocardiography ECG data, wherein the ECG data comprises abnormal heartbeat data; and
a second obtaining module (802), configured to obtain, based on the ECG data, an abnormal heartbeat waveform corresponding to the abnormal heartbeat data;
**characterized in that** the apparatus further comprises:
a combination module (803), configured to combine a normal heartbeat waveform and the abnormal heartbeat waveform to obtain an abnormality comparison image, wherein the abnormality comparison image displays a difference between the abnormal heartbeat waveform and the normal heartbeat waveform; and
a first generation module (804), configured to generate a diagnosis report based on the abnormality comparison image.

11. The apparatus according to claim 10, wherein the second obtaining module (802) is further configured to: determine whether heartbeat data in each heartbeat cycle in the ECG data is abnormal; and for each heartbeat cycle, obtain an abnormal heartbeat waveform in the heartbeat cycle if the heartbeat data in the heartbeat cycle is abnormal.

12. The apparatus according to claim 11, wherein the second obtaining module (802) is further configured to: obtain a first prevalence probability of each of a plurality of arrhythmia diseases based on the ECG data; obtain a second prevalence probability of each of the plurality of arrhythmia diseases based on the heartbeat data in each heartbeat cycle; perform calculation based on a plurality of first prevalence probabilities, a plurality of second prevalence probabilities, and preset probability weights, to obtain an overall prevalence probability of each arrhythmia disease; and determine, based on a plurality of overall prevalence probabilities, whether the heartbeat data in each heartbeat cycle is abnormal.

13. The apparatus according to claim 12, wherein the second obtaining module (802) is further configured to: extract a feature between heartbeat data in all heartbeat cycles in the ECG data, to obtain first feature data; and input the first feature data into a preset first classification model, to obtain the plurality of first prevalence probabilities; and
the second obtaining module (802) is further configured to: extract a feature of the heartbeat data in each heartbeat cycle, to obtain second feature data of the heartbeat data in each heartbeat cycle; and input each piece of second feature data into a preset second classification model, to obtain the plurality of second prevalence probabilities in the heartbeat data in each heartbeat cycle.

14. The apparatus according to claim 11, wherein the apparatus further comprises:
an addition module (805), configured to: for each heartbeat cycle, add a heartbeat waveform corresponding to the heartbeat cycle to a normal heartbeat waveform set if the heartbeat data in the heartbeat cycle is normal; and
a second generation module (806), configured to generate the normal heartbeat waveform based on at least one heartbeat waveform in the normal heartbeat waveform set.

15. A computer-readable storage medium having stored thereon the computer program according to any one of claims 1 to 9.

## Patentansprüche

1. Computerprogramm, umfassend Anweisungen, die, wenn das Programm durch einen Computer ausgeführt wird, den Computer veranlassen, Folgendes durchzuführen:
Erlangen (301, 401) von Elektrokardiografie- bzw. ECG-Daten;
Bestimmen (402), dass die ECG-Daten anormale Herzschlagdaten umfassen; und
Erlangen (302), basierend auf den ECG-Daten, einer anormalen Herzschlag-Wellenform korrespondierend mit den anormalen Herzschlagdaten;
**dadurch gekennzeichnet, dass** das Computerprogramm ferner den Computer veranlasst, Folgendes durchzuführen:
Kombinieren (303, 406) einer normalen Herzschlag-Wellenform und der anormalen Herzschlag-Wellenform, um ein Anomalie-Vergleichsbild zu erlangen, wobei das Anomalie-Vergleichsbild eine Differenz zwischen der anormalen Herzschlag-Wellenform und der normalen Herzschlag-Wellenform anzeigt; und
Erzeugen (304, 407) eines Diagnoseberichts basierend auf dem Anomalie-Vergleichsbild.

2. Computerprogramm nach Anspruch 1, wobei das Erlangen (302), basierend auf den ECG-Daten, einer anormalen Herzschlag-Wellenform korrespondierend mit den anormalen Herzschlagdaten Folgendes umfasst:
Bestimmen (403), ob Herzschlagdaten in jedem Herzschlagzyklus in den ECG-Daten anormal sind; und
für jeden Herzschlagzyklus Erlangen (404) einer anormalen Herzschlag-Wellenform in dem Herzschlagzyklus, wenn die Herzschlagdaten in dem Herzschlagzyklus anormal sind.

3. Computerprogramm nach Anspruch 2, wobei das Bestimmen (403), ob Herzschlagdaten in jedem Herzschlagzyklus in den ECG-Daten anormal sind, Folgendes umfasst:
Erlangen einer ersten Prävalenzwahrscheinlichkeit jeder einer Vielzahl von Arrhythmie-Erkrankungen basierend auf den ECG-Daten;
Erlangen einer zweiten Prävalenzwahrscheinlichkeit jeder der Vielzahl von Arrhythmie-Erkrankungen basierend auf den Herzschlagdaten in jedem Herzschlagzyklus;
Durchführen einer Berechnung basierend auf einer Vielzahl von ersten Prävalenzwahrscheinlichkeiten, einer Vielzahl von zweiten Prävalenzwahrscheinlichkeiten und im Voraus eingestellten Wahrscheinlichkeitsgewichtungen, um eine insgesamte Prävalenzwahrscheinlichkeit jeder Arrhythmie-Erkrankung zu erlangen; und
Bestimmen, basierend auf einer Vielzahl von insgesamten Prävalenzwahrscheinlichkeiten, ob die Herzschlagdaten in jedem Herzschlagzyklus anormal sind.

4. Computerprogramm nach Anspruch 3, wobei das Erlangen einer ersten Prävalenzwahrscheinlichkeit jeder einer Vielzahl von Arrhythmie-Erkrankungen basierend auf den ECG-Daten Folgendes umfasst:
Extrahieren eines Merkmals zwischen Herzschlagdaten in allen Herzschlagzyklen in den ECG-Daten, um erste Merkmalsdaten zu erlangen; und
Eingeben der ersten Merkmalsdaten in ein im Voraus eingestelltes erstes Klassifizierungsmodell, um die Vielzahl von ersten Prävalenzwahrscheinlichkeiten zu erlangen; und
wobei das Erlangen einer zweiten Prävalenzwahrscheinlichkeit jeder der Vielzahl von Arrhythmie-Erkrankungen basierend auf den Herzschlagdaten in jedem Herzschlagzyklus Folgendes umfasst:
Extrahieren eines Merkmals der Herzschlagdaten in jedem Herzschlagzyklus, um zweite Merkmalsdaten der Herzschlagdaten in jedem Herzschlagzyklus zu erlangen; und
Eingeben jedes Teils von zweiten Merkmalsdaten in ein im Voraus eingestelltes zweites Klassifizierungsmodell, um die Vielzahl von zweiten Prävalenzwahrscheinlichkeiten in den Herzschlagdaten in jedem Herzschlagzyklus zu erlangen.

5. Computerprogramm nach Anspruch 2, wobei das Computerprogramm ferner den Computer veranlasst, Folgendes durchzuführen:
für jeden Herzschlagzyklus Hinzufügen (405) einer Herzschlag-Wellenform korrespondierend mit dem Herzschlagzyklus zu einem Satz von normalen Herzschlag-Wellenformen, wenn die Herzschlagdaten in dem Herzschlagzyklus normal sind; und
Erzeugen (405) der normalen Herzschlag-Wellenform basierend auf mindestens einer Herzschlag-Wellenform in dem Satz von normalen Herzschlag-Wellenformen.

6. Computerprogramm nach Anspruch 5, wobei das Erzeugen (405) der normalen Herzschlag-Wellenform basierend auf mindestens einer Herzschlag-Wellenform in dem Satz von normalen Herzschlag-Wellenformen Folgendes umfasst:
Erlangen von Merkmalsdaten jedes Bands jeder Herzschlag-Wellenform in dem Satz von normalen Herzschlag-Wellenformen;
Eingeben der Merkmalsdaten jedes Bands in ein Wellenform-Korrekturmodell korrespondierend mit jedem Band, um eine Standard-Wellenform jedes Bands zu erlangen; und
Kombinieren einer Vielzahl von Standard-Wellenformen, um die normale Herzschlag-Wellenform zu erlangen.

7. Computerprogramm nach Anspruch 2, wobei das Computerprogramm ferner den Computer veranlasst, Folgendes durchzuführen:
Verwenden einer im Voraus eingestellten Elektrokardiogramm-Wellenform als die normale Herzschlag-Wellenform, wenn die Herzschlagdaten in jedem Herzschlagzyklus in den ECG-Daten anormal sind.

8. Computerprogramm nach Anspruch 2, wobei vor dem Bestimmen (403), ob Herzschlagdaten in jedem Herzschlagzyklus in den ECG-Daten anormal sind, das Computerprogramm ferner den Computer veranlasst, Folgendes durchzuführen:
Bestimmen, ob die ECG-Daten Arrhythmiedaten sind; und
wobei das Bestimmen, ob Herzschlagdaten in jedem Herzschlagzyklus in den ECG-Daten anormal sind, Folgendes umfasst:
wenn die ECG-Daten die Arrhythmiedaten sind, Bestimmen, ob die Herzschlagdaten in jedem Herzschlagzyklus in den ECG-Daten anormal sind.

9. Computerprogramm nach einem der Ansprüche 1 bis 8, wobei das Kombinieren (303, 406) einer normalen Herzschlag-Wellenform und der anormalen Herzschlag-Wellenform, um ein Anomalie-Vergleichsbild zu erlangen, Folgendes umfasst:
Ausrichten der anormalen Herzschlag-Wellenform mit der normalen Herzschlag-Wellenform basierend auf einer Zeitachse; und
Anzeigen der ausgerichteten anormalen Herzschlag-Wellenform und der ausgerichteten normalen Herzschlag-Wellenform basierend auf einer Achse derselben Amplitude, um das Anomalie-Vergleichsbild zu erlangen.

10. Diagnosebericht-Erzeugungsvorrichtung, umfassend:
ein erstes Erlangungsmodul (801), konfiguriert zum Erlangen von Elektrokardiografie- bzw. ECG-Daten, wobei die ECG-Daten anormale Herzschlagdaten umfassen; und
ein zweites Erlangungsmodul (802), konfiguriert zum Erlangen, basierend auf den ECG-Daten, einer anormalen Herzschlag-Wellenform korrespondierend mit den anormalen Herzschlagdaten;
**dadurch gekennzeichnet, dass** die Vorrichtung ferner Folgendes umfasst:
ein Kombinationsmodul (803), konfiguriert zum Kombinieren einer normalen Herzschlag-Wellenform und der anormalen Herzschlag-Wellenform, um ein Anomalie-Vergleichsbild zu erlangen, wobei das Anomalie-Vergleichsbild eine Differenz zwischen der anormalen Herzschlag-Wellenform und der normalen Herzschlag-Wellenform anzeigt; und
ein erstes Erzeugungsmodul (804), konfiguriert zum Erzeugen eines Diagnoseberichts basierend auf dem Anomalie-Vergleichsbild.

11. Vorrichtung nach Anspruch 10, wobei das zweite Erlangungsmodul (802) ferner konfiguriert ist zum: Bestimmen, ob Herzschlagdaten in jedem Herzschlagzyklus in den ECG-Daten anormal sind; und für jeden Herzschlagzyklus Erlangen einer anormalen Herzschlag-Wellenform in dem Herzschlagzyklus, wenn die Herzschlagdaten in dem Herzschlagzyklus anormal sind.

12. Vorrichtung nach Anspruch 11, wobei das zweite Erlangungsmodul (802) ferner konfiguriert ist zum: Erlangen einer ersten Prävalenzwahrscheinlichkeit jeder einer Vielzahl von Arrhythmie-Erkrankungen basierend auf den ECG-Daten; Erlangen einer zweiten Prävalenzwahrscheinlichkeit jeder der Vielzahl von Arrhythmie-Erkrankungen basierend auf den Herzschlagdaten in jedem Herzschlagzyklus; Durchführen einer Berechnung basierend auf einer Vielzahl von ersten Prävalenzwahrscheinlichkeiten, einer Vielzahl von zweiten Prävalenzwahrscheinlichkeiten und im Voraus eingestellten Wahrscheinlichkeitsgewichtungen, um eine insgesamte Prävalenzwahrscheinlichkeit jeder Arrhythmie-Erkrankung zu erlangen; und Bestimmen, basierend auf einer Vielzahl von insgesamten Prävalenzwahrscheinlichkeiten, ob die Herzschlagdaten in jedem Herzschlagzyklus anormal sind.

13. Vorrichtung nach Anspruch 12, wobei das zweite Erlangungsmodul (802) ferner konfiguriert ist zum: Extrahieren eines Merkmals zwischen Herzschlagdaten in allen Herzschlagzyklen in den ECG-Daten, um erste Merkmalsdaten zu erlangen; und Eingeben der ersten Merkmalsdaten in ein im Voraus eingestelltes erstes Klassifizierungsmodell, um die Vielzahl von ersten Prävalenzwahrscheinlichkeiten zu erlangen; und
wobei das zweite Erlangungsmodul (802) ferner konfiguriert ist zum: Extrahieren eines Merkmals der Herzschlagdaten in jedem Herzschlagzyklus, um zweite Merkmalsdaten der Herzschlagdaten in jedem Herzschlagzyklus zu erlangen; und Eingeben jedes Teils von zweiten Merkmalsdaten in ein im Voraus eingestelltes zweites Klassifizierungsmodell, um die Vielzahl von zweiten Prävalenzwahrscheinlichkeiten in den Herzschlagdaten in jedem Herzschlagzyklus zu erlangen.

14. Vorrichtung nach Anspruch 11, wobei die Vorrichtung ferner Folgendes umfasst:
ein Hinzufügungsmodul (805), konfiguriert zum: für jeden Herzschlagzyklus Hinzufügen einer Herzschlag-Wellenform korrespondierend mit dem Herzschlagzyklus zu einem Satz von normalen Herzschlag-Wellenformen, wenn die Herzschlagdaten in dem Herzschlagzyklus normal sind; und
ein zweites Erzeugungsmodul (806), konfiguriert zum Erzeugen der normalen Herzschlag-Wellenform basierend auf mindestens einer Herzschlag-Wellenform in dem Satz von normalen Herzschlag-Wellenformen.

15. Computerlesbares Speichermedium, auf dem das Computerprogramm nach einem der Ansprüche 1 bis 9 gespeichert ist.

## Revendications

1. Programme informatique comprenant des instructions qui, lorsque le programme est exécuté par un ordinateur, amènent l'ordinateur à effectuer :
l'obtention (301, 401) de données d'électrocardiographie ECG ;
la détermination (402) que les données ECG comprennent des données de battement de coeur anormal ; et
l'obtention (302), sur la base des données ECG, d'une forme d'onde de battement de coeur anormal correspondant aux données de battement de coeur anormal ;
**caractérisé en ce que** le programme informatique amène en outre l'ordinateur à effectuer :
la combinaison (303, 406) d'une forme d'onde de battement de coeur normal et de la forme d'onde de battement de coeur anormal pour obtenir une image de comparaison d'anomalie, l'image de comparaison d'anomalie affichant une différence entre la forme d'onde de battement de coeur anormal et la forme d'onde de battement de coeur normal ; et
la génération (304, 407) d'un rapport de diagnostic basé sur l'image de comparaison d'anomalie.

2. Programme informatique selon la revendication 1, l'obtention (302), sur la base des données ECG, d'une forme d'onde de battement de coeur anormal correspondant aux données de battement de coeur anormal comprenant :
la détermination (403) si des données de battement de coeur dans chaque cycle de battement de coeur dans les données ECG sont anormales ; et
pour chaque cycle de battement de coeur, l'obtention (404) d'une forme d'onde de battement de coeur anormal dans le cycle de battement de coeur si les données de battement de coeur dans le cycle de battement de coeur sont anormales.

3. Programme informatique selon la revendication 2, la détermination (403) de l'anomalie de données de battement de coeur dans chaque cycle de battement de coeur dans les données ECG comprenant :
l'obtention d'une première probabilité de prévalence de chacune d'une pluralité de maladies arythmiques sur la base des données ECG ;
l'obtention d'une deuxième probabilité de prévalence de chacune de la pluralité de maladies arythmiques sur la base des données de battement de coeur dans chaque cycle de battement de coeur ;
la réalisation d'un calcul basé sur une pluralité de premières probabilités de prévalence, d'une pluralité de deuxièmes probabilités de prévalence et de pondérations de probabilité prédéfinies, afin d'obtenir une probabilité de prévalence globale de chaque maladie arythmique ; et
la détermination, sur la base d'une pluralité de probabilités de prévalence globale, si les données de battement de coeur dans chaque cycle de battement de coeur sont anormales.

4. Programme informatique selon la revendication 3, l'obtention d'une première probabilité de prévalence de chacune d'une pluralité de maladies arythmiques sur la base des données ECG comprenant :
l'extraction d'une caractéristique entre des données de battement de coeur dans tous les cycles de battement de coeur dans les données ECG, afin d'obtenir des premières données de caractéristiques ; et
l'entrée des premières données de caractéristiques dans un premier modèle de classification prédéfini, afin d'obtenir la pluralité de premières probabilités de prévalence ; et
l'obtention d'une deuxième probabilité de prévalence de chacune de la pluralité de maladies arythmiques sur la base des données de battement de coeur dans chaque cycle de battement de coeur comprenant :
l'extraction d'une caractéristique des données de battement de coeur dans chaque cycle de battement de coeur, afin d'obtenir des deuxièmes données de caractéristiques des données de battement de coeur dans chaque cycle de battement de coeur ; et
l'entrée de chaque élément de deuxièmes données de caractéristiques dans un deuxième modèle de classification prédéfini, afin d'obtenir la pluralité de deuxièmes probabilités de prévalence dans les données de battement de coeur dans chaque cycle de battement de coeur.

5. Programme informatique selon la revendication 2, le programme informatique amenant en outre l'ordinateur à effectuer :
pour chaque cycle de battement de coeur, l'ajout (405) d'une forme d'onde de battement de coeur correspondant au cycle de battement de coeur à un ensemble de formes d'onde de battement de coeur normal si les données de battement de coeur dans le cycle de battement de coeur sont normales ; et
la génération (405) de la forme d'onde de battement de coeur normal sur la base d'au moins une forme d'onde de battement de coeur dans l'ensemble de formes d'onde de battement de coeur normal.

6. Programme informatique selon la revendication 5, la génération (405) de la forme d'onde de battement de coeur normal basée sur au moins une forme d'onde de battement de coeur dans l'ensemble de formes d'onde de battement de coeur normal comprenant :
l'obtention de données de caractéristiques de chaque bande de chaque forme d'onde de battement de coeur dans l'ensemble de formes d'onde de battement de coeur normal ; l'entrée des données de caractéristiques de chaque bande dans un modèle de correction de forme d'onde correspondant à chaque bande, afin d'obtenir une forme d'onde standard pour chaque bande ; et
la combinaison d'une pluralité de formes d'onde standard pour obtenir la forme d'onde de battement de coeur normal.

7. Programme informatique selon la revendication 2, le programme informatique amenant en outre l'ordinateur à effectuer :
l'utilisation d'une forme d'onde d'électrocardiogramme prédéfinie comme forme d'onde de battement de coeur normal si les données de battement de coeur dans chaque cycle de battement de coeur dans les données ECG sont anormales.

8. Programme informatique selon la revendication 2, avant la détermination (403) si des données de battement de coeur dans chaque cycle de battement de coeur dans les données ECG sont anormales, le programme informatique amenant en outre l'ordinateur à effectuer :
la détermination si les données ECG sont des données d'arythmie ; et
la détermination de l'anomalie des données de battement de coeur dans chaque cycle de battement de coeur dans les données ECG comprenant :
si les données ECG sont des données d'arythmie, la détermination si les données de battement de coeur dans chaque cycle de battement de coeur dans les données ECG sont anormales.

9. Programme informatique selon l'une quelconque des revendications 1 à 8, la combinaison (303, 406) d'une forme d'onde de battement de coeur normal et de la forme d'onde de battement de coeur anormal pour obtenir une image de comparaison d'anomalie comprenant :
l'alignement de la forme d'onde de battement de coeur anormal avec la forme d'onde de battement de coeur normal sur la base d'un axe temporel ; et
l'affichage de la forme d'onde de battement de coeur anormal alignée et de la forme d'onde de battement de coeur normal alignée sur un même axe d'amplitude, afin d'obtenir l'image de comparaison d'anomalie.

10. Appareil de génération de rapport de diagnostic, comprenant :
un premier module d'obtention (801), configuré pour obtenir des données d'électrocardiographie ECG, les données ECG comprenant des données de battement de coeur anormal ; et
un deuxième module d'obtention (802), configuré pour obtenir, sur la base des données ECG, une forme d'onde de battement de coeur anormal correspondant aux données de battement de coeur anormal ;
**caractérisé en ce que** l'appareil comprend en outre :
un module de combinaison (803), configuré pour combiner une forme d'onde de battement de coeur normal et la forme d'onde de battement de coeur anormal afin d'obtenir une image de comparaison d'anomalie, l'image de comparaison d'anomalie affichant une différence entre la forme d'onde de battement de coeur anormal et la forme d'onde de battement de coeur normal ; et
un premier module de génération (804), configuré pour générer un rapport de diagnostic sur la base de l'image de comparaison d'anomalie.

11. Appareil selon la revendication 10, le deuxième module d'obtention (802) étant en outre configuré pour : déterminer si des données de battement de coeur dans chaque cycle de battement de coeur dans les données ECG sont anormales ; et pour chaque cycle de battement de coeur, obtenir une forme d'onde de battement de coeur anormal dans le cycle de battement de coeur si les données de battement de coeur dans le cycle de battement de coeur sont anormales.

12. Appareil selon la revendication 11, le deuxième module d'obtention (802) étant en outre configuré pour : obtenir une première probabilité de prévalence de chacune d'une pluralité de maladies arythmiques sur la base des données ECG ; obtenir une deuxième probabilité de prévalence de chacune de la pluralité de maladies arythmiques sur la base des données de battement de coeur dans chaque cycle de battement de coeur ;
effectuer un calcul sur la base d'une pluralité de premières probabilités de prévalence, d'une pluralité de deuxièmes probabilités de prévalence et de pondérations de probabilité prédéfinies, afin d'obtenir une probabilité de prévalence globale de chaque maladie arythmique ; et déterminer, sur la base d'une pluralité de probabilités de prévalence globale, si les données de battement de coeur dans chaque cycle de battement de coeur sont anormales.

13. Appareil selon la revendication 12, le deuxième module d'obtention (802) étant en outre configuré pour : extraire une caractéristique entre des données de battement de coeur dans tous les cycles de battement de coeur dans les données ECG, pour obtenir des premières données de caractéristiques ; et entrer les premières données de caractéristiques dans un premier modèle de classification prédéfini pour obtenir la pluralité de premières probabilités de prévalence ; et
le deuxième module d'obtention (802) étant en outre configuré pour : extraire une caractéristique des données de battement de coeur dans chaque cycle de battement de coeur, afin d'obtenir des deuxièmes données de caractéristiques des données de battement de coeur dans chaque cycle de battement de coeur ; et entrer chaque élément de deuxièmes données de caractéristiques dans un deuxième modèle de classification prédéfini, afin d'obtenir la pluralité de deuxièmes probabilités de prévalence dans les données de battement de coeur dans chaque cycle de battement de coeur.

14. Appareil selon la revendication 11, l'appareil comprenant en outre :
un module d'addition (805), configuré pour : pour chaque cycle de battement de coeur, ajouter une forme d'onde de battement de coeur correspondant au cycle de battement de coeur à un ensemble de formes d'onde de battement de coeur normal si les données de battement de coeur dans le cycle de battement de coeur sont normales ; et
un deuxième module de génération (806), configuré pour générer la forme d'onde de battement de coeur normal sur la base d'au moins une forme d'onde de battement de coeur dans l'ensemble de formes d'onde de battement de coeur normal.

15. Support de stockage lisible par ordinateur sur lequel est stocké le programme informatique selon l'une quelconque des revendications 1 à 9.
